# EUROPÄISCHE PATENTANMELDUNG

(11) **EP 1 022 034 A1**
(43) Veröffentlichungstag der Anmeldung: **26.07.2000**
(21) Anmeldenummer: 99124821.2
(22) Anmeldetag: 14.12.1999
(51) Int. Cl.: A61N 1/36

(54) **Verfahren und Vorrichtung zur Stimulation von Muskeln oder Nervengewebe**

(30) Priorität: 19.01.1999 DE 19901872
(71) Anmelder: Leubner, Manfred, Dr., 94560 Offenberg (DE); Fischermeier, Martin, 94539 Grafling (DE); Kreter, Michael, 94474 Vilshofen (DE); Schosser, Johann, 94530 Auerbach (DE); Zelenka, Alois, 94136 Thyrnau (DE)
(72) Erfinder: Leubner, Manfred, Dr., 94560 Offenberg (DE); Fischermeier, Martin, 94539 Grafling (DE); Kreter, Michael, 94474 Vilshofen (DE); Schosser, Johann, 94530 Auerbach (DE); Zelenka, Alois, 94136 Thyrnau (DE)
(74) Vertreter: Schwabe - Sandmair - Marx

(57) **Zusammenfassung**

Die Erfindung bezieht sich auf ein Verfahren zum Erzeugen von elektrischen Impulssignalen zur Stimulation von Muskelgewebe und/oder Nervengewebe mit den Schritten: Erzeugen einer Anstiegsflanke eines Impulses, welche von einem 0-Wert auf den Impulsmaximalwert in einem Zeitraum von 30 µs bis 40 ms ansteigt; und Halten des Impulsmaximalwertes während einer Haltezeit, die etwa das 2,5-fache bis 9-fache der Dauer der Anstiegsflanke des Impulses beträgt. Weiter bezieht sich die Erfindung auf eine Vorrichtung zur Stimulation von Muskelgewebe und/oder Nervengewebe mit mindestens einer Kontaktelektrode, über welche elektrische Impulssignale an Muskelgewebe und/oder Nervengewebe angelegt werden können und einer Signalerzeugungsvorrichtung, welche mit der mindestens einen Kontaktelektrode verbunden ist, und elektrische Impulssignale erzeugt, wobei die Impulsdauer der Signale im Bereich zwischen 100 µs und 150 ms liegt, und die Anstiegszeit der Impulssignale etwa 10% bis 40% der Gesamtimpulsdauer beträgt. Ebenso bezieht sich die Erfindung auf die Verwendung der Vorrichtung zur Stimulierung von Muskelzellen bzw. Nervenzellen.

## Beschreibung

Die vorliegende Erfindung betrifft ein Verfahren und eine Vorrichtung zum Erzeugen von elektrischen Impulssignalen zur Stimulation von Muskel- und/oder Nervengewebe, sowie die Verwendung einer solchen Vorrichtung.

Vorrichtungen zum Erzeugen von elektrischen Impulssignalen zur Stimulation von Muskelgewebe bzw. Nervengewebe werden häufig zur Stimulation bzw. zum gezielten Aufbau von bestimmten Muskelgruppen eingesetzt, wie z.B. bei der Rehabilitation insbesondere älterer Menschen, denen eine gezielte Aktivierung gewisser Muskel- oder Nervengruppen mit zunehemenden Alter zusehends schwerer fällt.

Bei der Verwendung einer solchen Vorrichtung, welche häufig auch als Reizstrombehandlung bezeichnet wird, werden auf die Haut einer Person Kontaktelektroden angebracht, an die üblicherweise Stromimpulse mit Frequenzen von deutlich mehr als 50 Hz angelegt werden. Stromimpulse mit geeignet gewählter Amplitude bewirken eine Kontraktion von Muskelgruppen in der Nähe der Kontaktelektroden. Hierzu wird üblicherweise ein konstantes Frequenzmuster für die Stromimpulse verwendet.

Die Erfindung betrifft ferner die Verwendung einer elektromedizinischen Vorrichtung zur Verbesserung der körperlichen Erscheinung bzw. des Wohlbefindens einer erwachsenen, männlichen Person. Erwachsene, männliche Personen leiden zum Teil an einer erektilen Dysfunktion (mangelnde Versteifung des Gliedes), was zumeist Impotenz hervorruft (Unfähigkeit, den Geschlechtsverkehr zu vollziehen). Eine erektile Dysfunktion wird häufig durch eine Störung bzw. Schwäche des Muskulus corpus cavernosum hervorgerufen. Dieser Muskel leitet die Erektion (Versteifung) ein und unterhält sie, da er selektiv für die Füllung des Schwellkörpers sorgt, was für die Erektion unerläßlich ist.

Zur Behebung der erektilen Dysfunktion ist das gezielte Spritzen von Medikamenten in das Penisgewebe hinein bekannt, was jedoch mit Schmerzen und unerwünschten Nebenwirkungen, bis hin zur Dauererektion, und Unverträglichkeiten verbunden ist, die durch die bescheidene Erfolgsrate nicht gerechtfertigt erscheinen. Auch das Einsetzen von Implantaten in das Penisgewebe ist bekannt; diese führen jedoch häufig zu einer unerwünschten Narbenbildung und können nur schwer und unter erheblicher Narbenbildung wieder aus dem Penis entfernt werden.

Zur Behandlung ist hierzu vorab ein vertrauliches Gespräch mit einem Arzt oder Therapeuten über ein sehr intimes Thema unabdingbar, was bei den betroffenen Personen zumeist dazu führt, daß sie einem solchen Gespräch und somit auch einer Behandlung aus dem Wege gehen.

Beispielhaft für bekannte Vorrichtungen bzw. Verfahren zur elektrischen Stimulation von Muskeln wird auf die WO-A 93/24176, US 4,785,813, DE-OS 19 46 663 und DE 195 08 591 A1 verwiesen.

Dabei beziehen sich die DE-OS 19 46 663 und US 4,785,813 auf Geräte zur Steuerung der Muskeln von Lebenwesen, bei welchen ein elektromyographisches Signal, kurz EMG-Signal, von einem geeigneten Sensor abgetastet wird, um damit eine Muskelkontraktion zu detektieren. Nach dem Detektieren eines solchen EMG-Signals wird dann von der in diesen Druckschriften beschriebenen Vorrichtungen ein elektrisches Reizsignal auf entsprechende Muskelpartien übertragen, welche die Kontraktion z.B. der Muskeln am Blasenhals bewirken, so daß die Blase verschlossen wird. Die beschriebenen Vorrichtungen dienen also dazu eine Muskelkontraktion zu unterstützen, welche von einer Person nicht oder nicht vollständig durchgeführt werden kann, wobei das Ergebnis einer Steuerinformation, welche aus dem Gehirn über das Nervensystem zu dem betreffenden Muskel gelangt zunächst abgetastet wird.

Ein Nervenimpuls, welcher in dem zentralen Nervensystem erzeugt wird, depolarisiert dabei eine Membrane, welche eine kleine Gruppe von Muskelfasern umhüllt und welche als eine motorische Einheit bekannt ist. Diese motorische Einheit kontrahiert rasch und entspannt sich dann wieder, während andere gleichartige Einheiten gereizt werden. Das bei diesem Bewegungsprozeß entstehende EMG-Signal wird als Eingangssignal für die Ansteuerung der in diesen Druckschriften beschriebenen Vorrichtung verwendet. Dabei ist jedoch immer eine aktive Mitwirkung der Person erforderlich, d.h. diese Vorrichtungen können nicht dazu dienen eine bestimmte Muskelpartie einer Person zu stärken, ohne daß diese Person aktiv diese Muskelpartien ansteuert, z.B. wenn die betreffende Person schläft. Die oben genannten Druckschriften beziehen sich somit auf eine Vorrichtung, welche eine gewisse Art einer Verstärkung einer Kontraktion einer entsprechenden Muskelpartie bewirken kann.

Einen anderen Ansatz verfolgt die Vorrichtung gemäß der WO 93/24 176, mit welcher neuromuskuläre Erkrankungen bzw. Funktionsstörungen durch eine gezielte elektrische Stimulation behandelt werden sollten. Dazu ist eine Speichervorrichtung vorgesehen, welche ein auszugebendes Signalmuster abspeichern kann, wobei Impulse mit einer Rechteckform oder Dreiecks- bzw. Sägezahn-Form mit bestimmten elektrischen Randparamentern ausgegeben werden. Hierdurch können zwar bestimmte Muskelpartien gezielt gestärkt werden, jedoch hat sich in der Praxis gezeigt, daß mit den in dieser Druckschrift beschriebenen Signalformen und Parametern für die elektrischen Impulse nur vereinzelt Verbesserungen der entsprechenden Muskelpartien erhalten werden konnten.

In einer ähnlichen Ausgestaltung wie die Vorrichtung gemäß der oben beschriebenen WO 93/24176 bezieht sich die DE 195 08 591 A1 auf ein Gerät zur funktionellen Elektromyostimulation glatter Muskelzellen zur Therapie von Störungen der glatten Muskelzell-Funktion, insbesondere bei erektiler Dysfunktion und Inkontinenz bei Menschen und Säugetieren. Gemäß dieser Druckschrift werden die Stimulationsparameter so eingestellt, daß Nullliniensymetrische Impulse einer Rechteck- oder Trapezform mit einer Anstiegszeit von 0,01 bis 2 Sekunden, einer Stimulationsdauer von 1 bis 60 Sekunden gleichförmig oder alternierend, eine Stimulationspause von 0,01 bis 60 Sekunden, einem Frequenzbereich von 1 bis 50 Herz und eine Impulsdauer von 100 µs bis 10 ms verwendet werden. Wie bei dem vorab beschriebenen Gerät lassen sich hiermit vereinzelt Erfolge erzielen, wobei sich bei der praktischen Verwendung einer solchen Vorrichtung mit den oben angegebenen Parametern gezeigt hat, daß eine solche Behandlung von Muskelgewebe mit elektrischen Impulsen nur zu einer geringen bzw. nicht ausreichenden Stimulation von Muskelzellen führt.

Es ist die Aufgabe der vorliegenden Erfindung ein Verfahren und eine Vorrichtung zur Stimulation von Muskelgewebe und/oder Nervengewebe mit elektrischen Impulssignalen vorzuschlagen, welche die Nachteile des Standes der Technik verringern, insbesondere die Erfolgsquote verbessern. Weiterhin soll die Verwendung einer solchen Vorrichtung zur Verbesserung der körperlichen Erscheinung einer erwachsenen, männlichen Person vorgeschlagen werden, welche ebenfalls die Nachteile des Standes der Technik verringert. Insbesondere werden eine Vorrichtung und ein Verfahren vorgeschlagen, welche elektrische Impulssignale verwenden, wobei die elektrischen Impulse eine Trapezform aufweisen. Dabei ist die zeitliche Dauer der Anstiegsflanke eines solchen trapezförmigen Impulses auf die Dauer von etwa 30 µs bis 40 ms begrenzt, wobei nachfolgend auf diese Anstiegsflanke der Maximalwert des Impulses während einer Zeit gehalten wird, welche etwa das 1,5-fache bis 9-fache der Anstiegszeit der Flanke des trapezförmigen Impuls-Signals beträgt.

Es hat sich überraschend gezeigt, daß trapezförmige Impuls-Signale zur Stimulation von Muskelgewebe und/oder Nervengewebe besonders geeignet sind, wenn die gesamte Impulsdauer eines solchen trapezförmigen Einzelimpulses im Bereich von etwa 100 µs bis 150 ms liegt, wobei die Anstiegszeit des Impulssignals etwa 10 % bis 40% der gesamten Impulsdauer beträgt. Bei Versuchen hat sich ergeben, daß unter Verwendung einer Vorrichtung, welche die Impulse mit oben genannten Parametern erzeugt, überraschend eine erhebliche Verbesserung der Muskelstimulation bei Testpersonen erzielt werden konnte. Trapezförmige Impulse mit dem oben genannten Verhältnis der zeitlichen Dauer der Anstiegsflanke zu der gesamten Dauer des trapezförmigen Impulses haben sich als besonders wirksam zur Stimulation von Muskelpartien bzw. Nervengewebe erwiesen. Dabei kann der Impulsmaximalwert z.B. im Bereich von 0,5 mA bis 100 mA liegen.

Bevorzugt wird eine Abfolge von elektrischen Impulssignalen mit den oben beschriebenen elektrischen Randparametern so erzeugt, daß die einzelnen Impulse alternierend erzeugt werden, d.h. abwechselnd ein Impuls mit positiver und negativer Amplitude. Dabei ist es auch denkbar eine Gruppe von Impulsen mit einer positiven Amplitude zu erzeugen, worauf eine Gruppe von Impulsen mit einer negativen Amplitude folgt, usw. Es wird bevorzugt, daß die Impulssignale Wechselstromsignale ohne einen Gleichanteil darstellen, d.h. bezüglich der Nulllinie symmetrisch sind. Somit soll erreicht werden, daß bei einer längeren Behandlung von Muskelgewebe bzw. Nervengewebe kein Gleichstromanteil auftritt, welcher zu nicht gewünschten Hautrötungen oder ähnlichen unangenehmen Begleiterscheinungen führen kann. Das Ausgestalten der Abfolge der Impulssignale auf eine solche Art, daß kein Gleichstromanteil auftritt, bewirkt somit, daß keine elektrolytischen Reaktionen bei der Anwendung des Verfahrens auftreten können, da angelegte positive Spannungen bzw. Ladungen durch entsprechende bevorzugt symmetrische korrespondierende negative Spannungen bzw. Ladungen kompensiert werden. Allgemein ist es vorteilhaft eine geeignete Abfolge von elektrischen Impulsen mit oben genannten physikalischen Randparametern zu wählen, welche keinen Gleichstromanteil aufweisen, wobei diese Abfolge der Impulse nicht symmetrisch sein muß. So können z.B. für einen bestimmten Zeitraum eine Abfolge aus einem positiven und zwei negativen Impulsen verwendet werden, wobei darauf eine Abfolge von Impulsen über einen zweiten bestimmten Zeitraum mit umgekehrter Polarität folgt, um so bezüglich des Gleichstromanteils zu kompensieren.

Es hat sich als vorteilhaft erwiesen, das Austastsverhältnis der Impulse, d.h. z.B. das Verhältnis der Dauer eines positiven Einzelimpulses zum zeitlichen Abstand zu dem benachbarten negativen Impuls, klein zu halten, vorzugsweise kleiner als etwa 0,25, und weiter bevorzugt kleiner als 0,01. Auch hat sich ein Austastverhältnis von bis zu 10⁻⁴ als sehr wirkungsvoll zum gezielten Aufbau von Muskelgruppen erwiesen. Durch ein derartiges kleines Austastverhältnis wird die Ruhezeit der behandelten Nerven- bzw. Muskelpartien zwischen einem positiven und negativen Impuls relativ klein gehalten, so daß die entsprechenden Gewebezellen fast einer ununterbrochenen bzw. konstanten Beanspruchung bzw. Reizung zwischen den jeweiligen Impulsen unterliegen, um so den Wirkungsgrad der eingesetzten elektrischen Impulse zu verbessern. Dabei kann die Abfallzeit des Impulses relativ klein sein, so daß die abfallende Flanke des Impulses nahezu senkrecht verläuft, wobei auch sehr gute Ergebnisse mit einer Abfallzeit von bis zu 25% der Gesamtimpulsdauer erhalten wurden. Allgemein kann durch ein geeignetes Einstellen der Anstiegszeit bzw. Abfallzeit des Impulses die Geschwindigkeit der Kontraktion bzw. Entspannung eines entsprechenden Muskelgewebes eingestellt werden, um den Behandlungserfolg zu optimieren, wobei sich gezeigt hat, daß das Verhältnis von Anstiegszeit zur Gesamtdauer des Impulses für die Wirksamkeit entscheidend ist.

Es hat sich weiter gezeigt, daß je nach Art der zu behandelnden Muskelgewebe bzw. Nervengewebe unterschiedliche Betriebsarten, oft auch in Abhängigkeit von der zu behandelnden Person, unterschiedlich wirksam sein können. Eine effektive Möglichkeit einer solchen Betriebsart ist der Dauerfrequenzbetrieb, bei welchem Stromimpulse permanent bzw. ununterbrochen erzeugt und an die entsprechenden Muskel- bzw. Nervenzellenpartien angelegt werden. Dabei treten im wesentlichen keine Unterbrechungen der angelegten Impulse auf, mit Ausnahme des oben erwähnten Austastverhältnisses. Es kann jedoch auch vorteilhaft sein, die Impulse in einem sogenannten Intervall-Frequenzbetrieb anzulegen, wobei die Stromimpulse für einen ersten bestimmten Zeitraum, wie z.B. 6,4 Sekunden anliegen, und nachfolgend in einem zweiten Zeitraum, wie z.B. 10,6 Sekunden, keine Stromimpulse angelegt werden. Danach wird das Anlegen der Stromimpulse während des ersten Zeitraums wiederholt und so weiter. Die beispielhaft genannten Zeiträume sollen nicht beschränkend ausgelegt werden. Vielmehr ist es auch denkbar die Stromimpulse für einen längere Zeit anzulegen und den dazwischenliegenden Ruhezeitraum kürzer auszugestalten, also z.B. die Stromimpulse wären 10 Sekunden anzulegen, gefolgt von einem Ruhezeitraum von ca. 5 Sekunden. Allgemein ist es denkbar, Impulse zum Beispiel während ca. 2 bis 12 Sekunden anzulegen, gefolgt von ca. 2 bis 12 Sekunden Pause. Dabei können die Stromimpulse, wie oben beschrieben alternierend angelegt werden, d.h. auf einen positiven Impuls folgt ein negativer und umgekehrt, oder mit einer Abfolge von einem oder mehreren positiven Impulsen, auf welche eine oder mehrere negative Impulse folgen.

Weitere Betriebsarten haben sich je nach Einsatzgebiet und je nach zu behandelnder Person als vorteilhaft erwiesen wie z.B. die Verwendung von jeweils einem positiven und einem negativen Impuls mit jeweils einer Impulsdauer zwischen z.B. 30 ms und 150 ms, wobei dann z.B. mit einer Periodendauer von 500 ms, d.h. mit einer Frequenz von 2 Hz, die Sequenz bestehend aus positivem und negativem Impuls erneut angelegt wird. Ebenso hat es sich im Einzelfall als vorteilhaft erwiesen die Dauer eines einzelnen Impulses auf 100 µs festzulegen, wobei die Frequenz z.B. 50 Hz betragen kann, d.h. alle 20 ms werden die angelegten positiven und negativen Stromimpulse wiederholt.

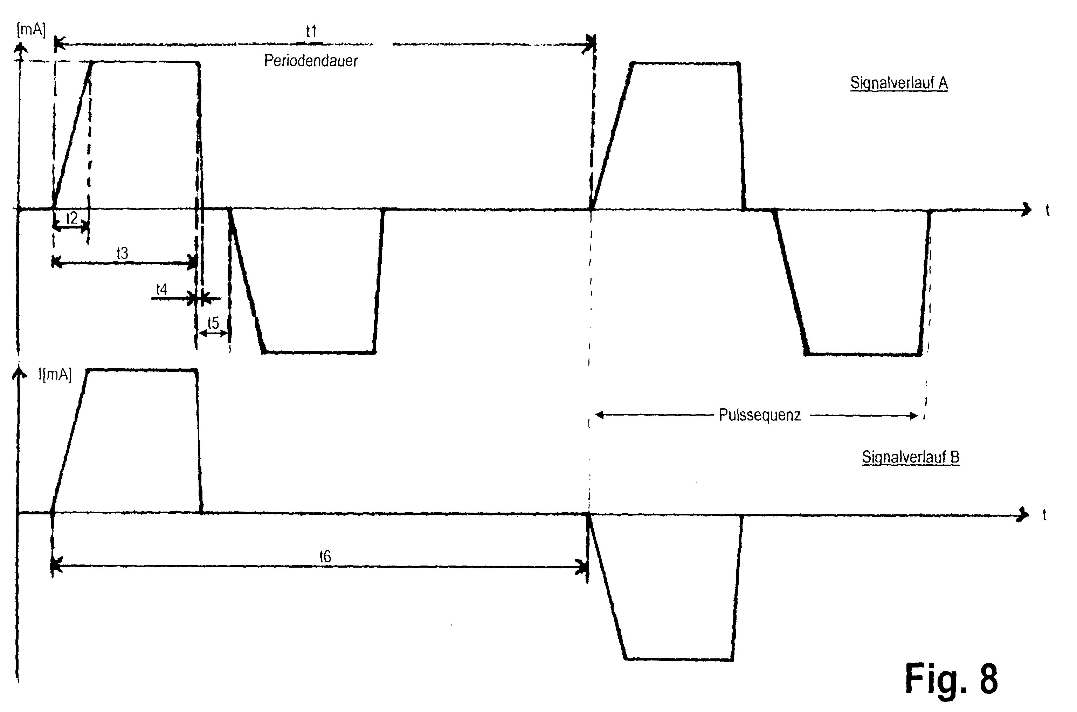

Ebenso hat es sich als vorteilhaft erwiesen, die Impulsdauer auf etwa 100 µs festzulegen, wobei die Periodendauer der Pulssequenzen, also der zeitliche Abstand zwischen dem Beginn, z.B. zweier positiver Impulse, zwischen 10 ms und 1 s gewählt wird; siehe z.B. Figur 8A. Dabei kann während einer Behandlung über eine längere Zeit die Periodendauer auch nach einem bestimmten Muster verändert werden, z.B. kontinuierlich erhöht bzw. verringert werden, um so eine vorteilhafte Stimulation von Muskelzellengewebe bzw. Nervenzellengewebe zu erzielen. Figur 8B zeigt ein alternatives Sinalmuster, wobei der Abstand zwischen einem positiven und negativen Impuls vergrößert wurde.

Allgemein kann die Verwendung von trapezförmigen Impulsen mit oben genannten Parametern so erfolgen, daß eine feste Spannungs- bzw. Stromimpulsdauer gewählt oder die Spannungsbzw. Stromimpulsdauer über die Zeit innerhalb der genannten Grenzen variiert wird, unter Berücksichtigung des oben angegebenen Verhältnisses, d.h. die Anstiegszeit der Impulsflanke beträgt etwa 10% bis 40% der gesamten Impulsdauer. Dabei kann eine Umpolarisation der Impulse mit einer festen oder auch frei wählbaren Frequenz erfolgen.

Besonders vorteilhaft ist es, die Anstiegszeit des Impulssignals so zu wählen, daß sie etwa 20% bis 30%, bevorzugt ca. 25%, der gesamten Impulsdauer beträgt, so daß im Falle der Dauer der Anstiegszeit von 25% das Signal mit seiner Maximalamplitude etwa dreimal so lang anliegt, wie das Signal benötigt hat, um auf die Maximalamplitude von 0 beginnend anzusteigen. Die Gesamtimpulsdauer liegt dabei jeweils bevorzugt im Bereich von 100 µs bis 150 ms. Es wird davon ausgegangen, daß die abfallende Flanke sehr steil ist, wobei natürlich, wie oben erwähnt, die Dauer der abfallenden Flanke ebenso bis zu 25% der gesamten Impulsdauer betragen kann. Die steigende sowie die abfallende Flanke sind vorteilhaft in etwa Geraden, wobei auch parabelförmige oder anders kurvenförmig verlaufende Flanken möglich sind. Es wird angenommen, daß die vorteilhafte Wirkung der Impulssignale mit den genannten elektrischen Parametern darauf beruht, daß der Trainingseffekt eines Muskels bzw. die Reizung eines Nervengewebes besonders effektiv ist, wenn der Impulsanstieg auf etwa 1/4 der gesamten Dauer des Impulses festgelegt wird, worauf das entsprechende Muskelgewebe bzw. Nervengewebe einer in etwa konstanten Reizung mit etwa der dreifachen Dauer der Anstiegszeit des Impulses, bei einer Maximalamplitude des Impulses ausgesetzt wird.

Die erfindungsgemäße Vorrichtung zur Stimulation von Muskelgewebe und/oder Nervengewebe umfaßt mindestens eine, vorzugsweise zwei, Kontaktelektroden, über welche elektrische Signale an ein Muskelgewebe und/oder Nervengewebe angelegt werden können, wodurch das Muskel- und/oder Nervengewebe funktionell stimuliert wird. Weiterhin ist eine Signalerzeugungsvorrichtung vorgesehen, welche mit der bzw. den Kontaktelektroden in Verbindung steht, und elektrische Impulssignale erzeugt. Diese Impulssignale können sowohl Stromsignale als auch Spannungssignale sein, so daß die entsprechenden Spannungs- bzw. Stromsignale über die Kontaktelektroden an entsprechende Muskel- bzw. Nervenpartien angelegt werden können. Die Signalerzeugungsvorrichtung erzeugt dabei Impulssignale mit einer Impulsdauer im Bereich von 100 µs bis 150 ms. Die erzeugten Impulse sind dabei vorzugsweise trapezförmig. Die Anstiegszeit des Impulssignals auf den maximalen Amplitudenwert des Impulses liegt dabei im Bereich zwischen 10% und 40% der gesamten Impulsdauer, vorzugsweise 20% bis 30%, insbesondere 25%, so daß im letzteren Falle die Maximalamplitude ca. 75% der Gesamtimpulsdauer anliegt. Mit einer solchen Vorrichtung können die oben beschriebenen vorteilhaften Wirkungen erreicht werden.

Bevorzugt ist bei der erfindungsgemäßen Vorrichtung eine oder mehrere Einstellvorrichtungen vorgesehen, mit welchen bevorzugt stufenlos bestimmte Parameter des Impulssignals bzw. einer Abfolge von Impulssignalen eingestellt werden können. Insbesondere ist es vorteilhaft eine Einstellvorrichtung zum Einstellen der Intensität eines Stromimpulses bzw. Spannungsimpulses vorzusehen, mit welchem die Maximalamplitude des Impulses eingestellt werden kann. So hat es sich z.B. als vorteilhaft erwiesen im Fall von angelegten Spannungssignalen die Amplitude in einen Bereich zwischen 0 V und 160 V einstellbar auszugestalten. Dabei ist jedoch zu berücksichtigen, daß es im Wesentlichen auf den eingeprägten Strom ankommt. Eine solche Einstellvorrichtung kann ein Drehregler sein, wie z.B. ein Potentiometer, welches bevorzugt auch zum Einschalten der gesamten Vorrichtung verwendet wird, so daß nach dem Einschalten nicht sofort ein Maximalwert anliegt, sondern beginnend von einem niedrigeren Anfangswert der Maximalamplitude der eingestellte Wert der Maximalamplitude langsam erhöht wird. Dabei ist es auch denkbar, die Vorrichtung so auszugestalten, daß erst eine gewisse Zeitdauer nach dem Einschalten, wie z.B. 6 Sekunden, kein Signal anliegt und nachfolgend erst mit dem Ausgeben der Impulse begonnen wird. Weiterhin ist es vorteilhaft die Gesamtdauer des Impulses einstellbar auszugestalten, und zwar in einem Bereich zwischen 100 µs und 150 ms, wobei vorteilhaft die Anstiegszeit des Impulses ebenso separat eingestellt werden kann, so daß sie bei etwa 10% bis 40% der gesamten Impulsdauer des Impulssignales liegt. Ebenso ist es vorteilhaft eine weitere Einstellvorrichtung vorzusehen, über welche eine Periodendauer der Impulse eingestellt werden kann, d.h., daß z.B. eine Frequenz zwischen 1 Hz und 100 Hz gewählt werden kann, so daß eine Umpolarisation der ausgegebenen aufeinander folgenden Einzelimpulse alle 500 ms oder 10 ms, bzw. bei einem geeigneten Zwischenwert erfolgt. Ebenso kann vorteilhaft eine Einstellvorrichtung zur Einstellung des Austastverhältnisses der Impulse vorgesehen sein.

Bei einer erfindungsgemäßen Ausführungsform kann mit der erfindungsgemäßen Vorrichtung gekoppelt an einen Eingang der Vorrichtung eine Meßelektrode bzw. Meßsonde angeschlossen sein, welche dem Abgriff eines Nervenimpulses bzw. Spannungssignals von einer Körperzelle des Benutzers mit einer darunter befindlichen Muskel- und/oder Nervenpartie dient. Dieser abgetastete Nervenimpuls wird vom Benutzer vorzugsweise intentionsabhängig zur Aktivierung oder Ausführung einer körperlichen Funktion erzeugt. Eine bei der Vorrichtung geeignet vorgesehene Triggerfunktion kann dabei vorteilhaft eine intentionsabhängige Steuerung des Gerätes bewirken. D.h. der Benutzer kann über eine willkürlich verursachte Muskelbewegung bewirken, daß ein einzelner Impuls bzw. eine vorher spezifizierte Abfolge mehrerer Impulse von der erfindungsgemäßen Vorrichtung ausgegeben wird, wenn eine solche Muskelbewegung des Benutzers von der Meßelektrode bzw. Meßsonde detektiert wird. Jedoch kann der abgegriffene Nervenimpuls von der Person auch unbewußt erzeugt werden, wobei dann mit Hilfe der genannten Triggerfunktion der erfindungsgemäßen Vorrichtung ein geeigneter Impuls bzw. eine Abfolge mehrerer Impulse ausgegeben wird, um einen gewünschten Effekt im Körper des Benutzers, d.h. bei den entsprechenden Nervenzellen bzw. Muskelzellen, auszulösen. Ein von der Vorrichtung erzeugter Stromimpuls oder Spannungsimpuls, bzw. eine Abfolge davon, dient dann der Aktivierung oder Ausführung der körperlichen Funktion des Benutzers.

Dabei kann bei einem solchen Triggerbetrieb der erfindungsgemäßen Vorrichtung der abgegriffene Spannungs- bzw. Nervenimpuls in der Vorrichtung geeignet als Signal aufbereitet werden, wie z.B. verstärkt und/oder gefiltert werden, bevor er als Triggersignal zur Initiierung einer Aktion des Gerätes verwendet wird. Weiterhin kann ein geeigneter Schwellenwert vorgegeben werden, welcher nach Überschreiten durch das abgegriffene und ggfs. gefilterte bzw. verstärkte Spannungssignal bewirkt, daß die Vorrichtung einen bestimmten Impuls bzw. eine Impulsfolge ausgibt, welche über Kontaktelektroden zur Stimulation von Muskel- und/oder Nervengewebe verwendet werden kann.

Je nach Verwendung der erfindungsgemäßen Vorrichtung kann es vorteilhaft sein mehr als nur eine Abtastelektrode bzw. Meßelektrode vorzusehen, um die entsprechenden Muskel- bzw. Nervensignale des Benutzers aufzufangen, und anschließend können über eine oder mehr Elektroden die zur Stimulation ausgegebenen Impulse auf den Benutzer übertragen werden. Es ist auch denkbar ein und dieselbe Elektrode bzw. ein Elektrodenpaar oder auch mehrere Elektroden so zu schalten, daß über diese Elektrode sowohl das Detektieren eines Nerven- oder Muskelsignals des Benutzers vorgenommen werden kann, als auch das nachfolgende Abgeben bzw. Übertragen von elektrischen Impulsen auf die entsprechenden Nerven- bzw. Muskelzellen.

Vorteilhaft weist die erfindungsgemäße Vorrichtung eine Anordnung zur Hochspannungserzeugung auf, welche die erforderliche Spannung erzeugt, um den gewünschten Strom in den menschlichen Körper einprägen zu können. Dazu können bekannte Wandler verwendet werden, wie z.B. ein Eintakt-Sperrwandler oder ein Eintakt-Durchflußwandler.

Die erfindungsgemäße Vorrichtung kann vorteilhaft so ausgestaltet sein, daß die einzelnen Betriebsarten durch eine entsprechende Veränderung der hardwaremäßigen Konfiguration fest eingestellt werden kann, wie z.B. dem Verändern eines geeigneten Überbrückungskontaktes bzw. Jumpers.

Die erfindungsgemäße Vorrichtung erzeugt die elektrischen Impulse bevorzugt so, daß diese keinen Gleichstromanteil aufweisen, so daß diese z.B. bei einem geeigneten Betrachtungszeitraum einen 0-Symmetrie aufweisen können. Insbesondere ist die erfindungsgemäße Signalerzeugungsvorrichtung so ausgestaltet, daß die oben beschriebenen Einzelimpulse bzw. Abfolgen von Impulsen mit ebenfalls oben beschriebener Umpolarisation durchgeführt werden können. Dabei ist es vorteilhaft mehrere Betriebsarten, wie oben beschrieben, fest vorzugeben, wobei dann die entsprechenden Parameterisierungen der einzelnen Betriebsarten, wie oben erwähnt, vorgenommen werden können. Vorteilhaft ist die erfindungsgemäße Vorrichtung netzunabhängig und weist eine Energiequelle, wie z.B. eine Batterie oder einen Akku auf, so daß diese Vorrichtung für den mobilen Einsatz verwendet werden kann und von dem Benutzer ohne größere Einschränkungen in einem weiten Bereich verwendet werden kann.

Vorzugsweise sind Batterieeingang und Ausgang der Vorrichtung galvanisch getrennt. Weiterhin ist der Ausgang vorzugsweise verriegelungssicher, so daß bei Anschluß der Kontaktelektroden an das Gerät nicht sofort die gewählte Maximalamplitude an der Kontaktelektrode anliegt.

Allgemein umfaßt die erfindungsgemäße Vorrichtung eine Signalerzeugungsvorrichtung ,welche ein Ausgangssignal mit oben beschriebenen Randparametern erzeugt, ggfs. nach Verstärkung bzw. Filterung im Gerät an entsprechende Kontaktelektroden weiterleitet, welche auf einer Köperstelle einer Person angebracht werden, um für eine spezifische funktionelle Stimulation von Muskelgewebe und/oder Nervengewebe zu sorgen. Dabei kann eine lokale Muskelpartie bzw. Nervenpartie ausgewählt und mit einem geeigneten Betriebsmodus hinsichtlich einer gewünschten Wirkung auf den Organismus stimuliert bzw. trainiert werden.

Die erfindungsgemäße Vorrichtung ist vorteilhaft nach den Richtlinien für medizintechnischen Geräte DIN VDE 0750 Teil 219 ausgestaltet, so daß der Benutzer frei bei der Verwendung der Vorrichtung ist. Dies ist insbesondere vorteilhaft, da ein Benutzer dann zum Training bzw. zur Stimulation geeigneter Muskelzellen bzw. Nervenzellen in der Lage ist, ohne einen Arzt konsultieren zu müssen. Der Benutzer kann somit den Trainingsumfang oder die Stimulationsintensität den eigenen Bedürfnissen anpassen.
Erfindungsgemäß wird die Vorrichtung, wie oben beschrieben, zur gezielten Stimulation bzw. zum Training von Muskelgewebe und/oder Nervengewebe verwendet. Insbesondere wird die erfindungsgemäße Vorrichtung zur gezielten, lokalen Stimulation bzw. zum Aufbau des Muskulus corpus cavernosum bei einer erwachsenen, männlichen Person verwendet. Hierzu wird zumindest eine Kontaktelektrode in der Nähe der Peniswurzel angebracht. Ein geeignet auf diesen Muskel abgestimmtes Frequenzmuster wird von der Signalerzeugungsvorrichtung erzeugt und an die Kontaktelektrode abgegeben. Dadurch kommt es zu einer funktionellen Stimulation des Muskulus Corpus Cavernosum (Mcc), der vor und während des Geschlechtsverkehrs eine Füllung des Schwellkörpers bewirkt.

Bei einer möglichen Verwendung des Gerätes erfolgt die Stimulation unmittelbar vor und/oder während des Geschlechtsverkehrs, wobei die kosmetische Wirkung der Verwendung bzw. des Verfahrens zum Tragen kommt, nämlich aufgrund der verbesserten, weil männlicheren körperlichen Erscheinung der Person.

Bevorzugt wird der Mcc jedoch nicht zum Geschlechtsverkehr, sondern mehrmals täglich bzw. je nach den Bedürfnissen des Benutzers stimuliert, was zu einem gezielten Aufbau bzw. einer gezielten Stärkung dieses Muskels führt. Die besondere Wirksamkeit von Stromimpulsen mit den erfindungsgemäßen Parametern zur Stärkung des Mcc war bisher nicht bekannt.

Vorteilhaft ist ferner, daß es zum Geschlechtsverkehr keiner umständlichen Manipulation und keines Anlegens von Hilfsmittel jeglicher Art bedarf, da der so gestärkte Mcc automatisch zur erforderlichen Füllung des Schwellkörpers führt.

Vorteilhaft ist, daß aufgrund der netzunabhängigen Einsatzmöglichkeit des Gerätes mit Hilfe einer eigenen Spannungsversorgung diese Stimulation bei Bedarf vorgenommen werden kann, beispielsweise zu Hause, auf der Arbeit oder unterwegs, und nicht nur unmittelbar vor oder gar während des Geschlechtsverkehrs vorgenommen werden muß.
Die dadurch bewirkte verbesserte Erektionsfähigkeit führt bei erwachsenen, männlichen Personen im Bedarfsfall zu einer verbesserten körperlichen Erscheinung.

Weitere vorteilhafte Anwendungsformen des erfindungsgemäßen Gerätes, insbesondere in netzunabhängiger Betriebsweise, betreffen die Verbesserung der körperlichen Erscheinung einer Person durch gezielten Muskelaufbau durch Muskelstimulation z.B. nach einem Schlaganfall, durch gezielte Stimulation und Stärkung der Muskulatur bzw. des jeweiligen Schließmuskel der Blase oder des Mastdarms, damit es erst gar nicht zu einer Blasen- oder Mastdarminkontinenz kommt, und durch gezielte Stimulation des Musculus temporalis.

Bei einer bevorzugten Verwendung des erfindungsgemäßen Gerätes wird statt der streifenförmigen Kontaktelektroden ein zylinder- bzw. ellipsenförmiger Körper mit aufgesetzten, vorzugsweise zwei Streifenelektroden in Umfangsrichtung eingesetzt. Die beiden Streifenelektroden befinden sich hierzu erfindungsgemäß nicht am vorderen und hinteren Endbereich dieses Körpers, sondern in einem Mittelbereich, wobei der Abstand der beiden Elektroden geeignet an die zu stimulierende Muskel- bzw. Nervenpartie angepaßt ist und bevorzugt etwa 1 bis 2 cm entspricht.

Zur individuellen Anpassung des Elektroden-Körpers kann dieser in seinem Umfang variierbar sein, beispielsweise durch Aufpumpen, wobei die Elektroden dann vorzugsweise einen Umfangseinschnitt aufweisen und dehnbar ausgebildet sind. Damit der Körper nach Einführen nicht in der Körperöffnung verschwindet, ist der erfindungsgemäße Elektroden-Körper vorzugsweise an einem Ende mit einem Anschlag versehen, der bevorzugt als Gummiring ausgebildet ist, der in Axialrichtung geeignet verschoben werden kann, jedoch einer solchen Verschiebung einen geeigneten Widerstand entgegensetzt, so daß er der Anschlagsfunktion dienen kann. Vorteilhaft kann so eine individuelle Anpassung des Elektrodenkörpers vorgenommen werden.

Nachfolgend werden bevorzugte Ausführungsbeispiel der Erfindung unter Bezugnahme auf die beiliegende Figuren beschrieben. Dabei zeigen:
- Fig. 1: eine perspektivische Schemadarstellung eines erfindungsgemäßen Gerätes;
- Fig. 2: ein Blockschaltbild eines erfindungsgemäßen Gerätes, das die wesentlichen funktionsbeteiligten Elemente zusammenfaßt;
- Fig. 3: ein Funktionsschema zur Festlegung des Frequenzmusters eines erfindungsgemäßen Gerätes;
- Fig. 4: ein weiteres Funktionsschema zur Festlegung des Frequenzmusters eines erfindungsgemäßen Gerätes;
- Fig. 5: zwei bevorzugte Ausführungsformen von Elektrodenkörpern, die jeweils mit zwei Elektroden in Umfangsrichtung versehen sind;
- Fig. 6: einen beispielhaften Signalverlauf eines Impulses gemäß der vorliegenden Erfindung;
- Fig. 7: ein Blockschaltbild der erfindungsgemäßen Vorrichtung; und
- Fig. 8A und B: zwei zeitliche Abfolgen des erfindungsgemäßen Signalmusters.

Es wird darauf hingewiesen, daß diese Figuren und die entsprechende Beschreibung, wie nachfolgend dargelegt, nur in beispielhafter und nicht etwa nur in einer auf diese eine konkrete Ausführungsform beschränkten Art und Weise auszulegen sind.

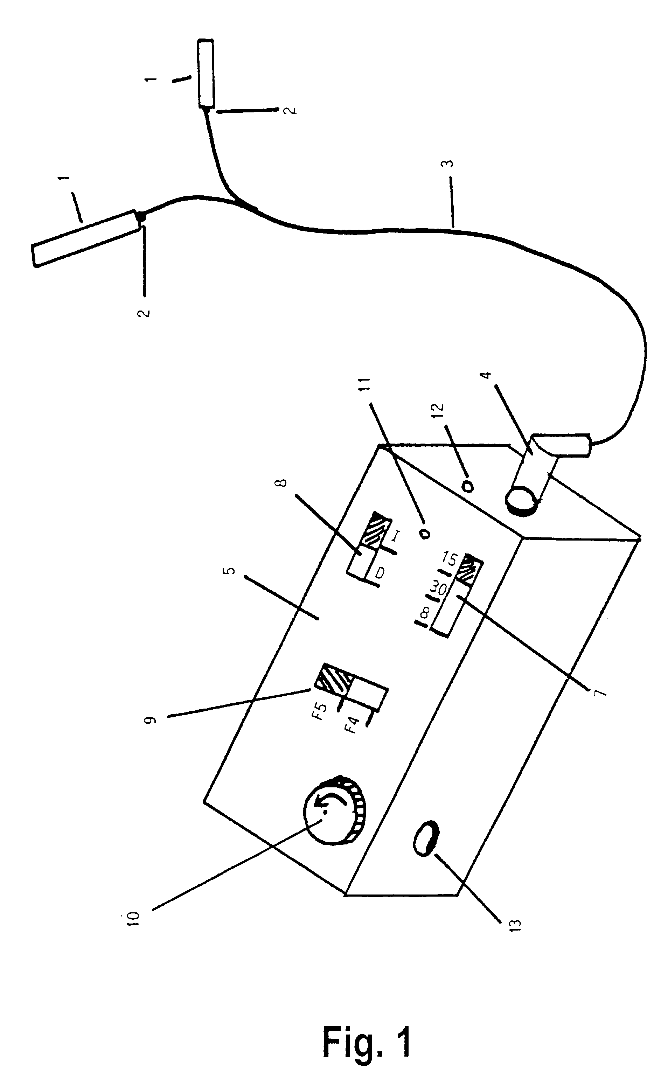

Fig. 1 zeigt eine perspektivische Schemadarstellung eines erfindungsgemäßen Gerätes.

In einem Gehäuse 5 sind als Signalerzeugungsvorrichtung ein Oszillator (nicht dargestellt) und eine netzunabhängige Spannungsversorgung (nicht dargestellt) wie etwa eine Batterie oder ein Akku mit vorzugsweise 9 Volt Betriebsspannung untergebracht. Hierdurch ist ein netzunabhängiger Betrieb möglich.
Das Gerät verfügt über einen Ausgang, vorzugsweise eine Buchse, an dem das Ausgangssignal des Oszillators anliegt. Der Oszillator dient der Erzeugung der erfindungsgemäßen Spannungs- oder Stromimpulse, wie nachfolgend noch beschrieben wird, und ist bevorzugt auf einer elektronischen Schaltkarte gemeinsam mit einer Ansteuerelektronik angeordnet.

Über einen speziell geformten Stecker, der aufgrund seiner Form nicht in eine Steckdosenöffnung paßt, was ein besonderes Sicherheitsmerkmal darstellt, und ein vorzugsweise abgeschirmtes Verbindungskabel 3 sind die Kontaktelektroden 1 mit dem Gerät verbunden. Ein Paar von Kontaktelektroden wird auf einen Hautbereich in der Nähe eines bestimmten Muskel- und/oder Nervengewebes angebracht. Bei anderen Verwendungszwecken kann jedoch auch eine andere Zahl von Kontaktelektroden verwendet werden. Bevorzugt werden die Kontaktelektroden symmetrisch um das zu stimulierende Muskel- und/oder Nervengewebe angeordnet.

Die Kontaktelektroden sind vorzugsweise über lösbare Kontakte 2 wie etwa eine Knopf- oder Steckverbindung mit dem Verbindungskabel 3 verbunden, so daß die Kontaktelektroden zur Einhaltung von hygienischen Mindeststandards, beispielsweise bei einer aufeinanderfolgenden Verwendung bei verschiedenen Personen, ausgetauscht oder ersetzt werden können.

Leuchtdioden zeigen jeweils an, daß das Gerät eingeschaltet ist (grüne LED 11) und gerade Stromimpulse ausgibt (gelbe LED 12). Bevorzugt ist der Geräteausgang verpolungssicher (z.B. über eine Dioden- oder Transistorenschaltung) und verfügt über eine Ausgangsverriegelung gemäß DIN VDE 0750, Teil 219, Unterpunkt 51.102. Beim Umschalten zwischen den Frequenzen kann z.B. automatisch ausgeschaltet werden.

Bevorzugt ist das Gerät ausgelegt, um bei der vorgesehen Betriebsspannung von z.B. 9 Volt bei einem Hautwiderstand von 5 kOhm eine maximale Ausgangsstromstärke von 40 mA auszugeben. Unterhalb von dieser maximalen Ausgangsstromstärke kann die Amplitude der Stromimpulse beliebig eingestellt werden, wodurch die Intensität der Stimulation variiert werden kann. Ferner ist das Gerät ausgelegt, daß gemäß DIN VDE 9750, Teil 219, Unterpunkt 51.104 der Stromgrenzwert von 60 mA und eine Ausgangsspannung von 500 V bei einem Lastwiderstand von 500 Ohm nicht überschritten werden.

Zur Beeinflussung des Betriebszustands verfügt das Gerät über ein Drehpotentiometer 10, vorzugsweise mit Rastfunktion, welches in einer Betriebsart (Dauerbetrieb, Intervallbetrieb) zur Regelung der Amplitude der Stromimpulse und in einer weiteren Betriebsart (Triggerbetrieb) zur Regelung einer geräteinternen Verstärkung, wie noch beschrieben wird, dient.

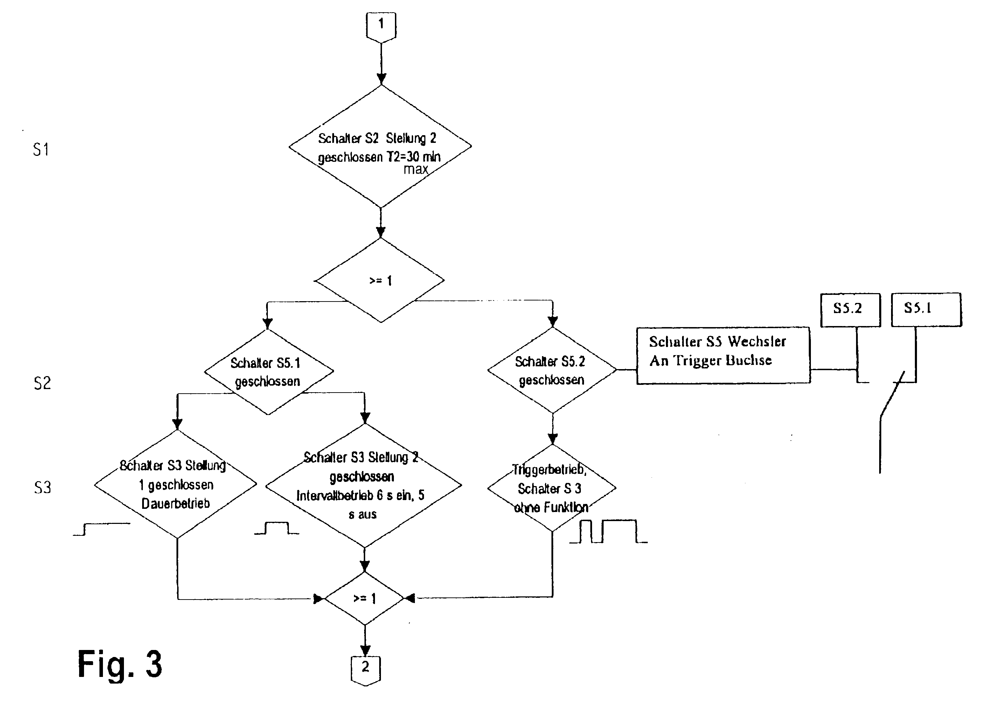

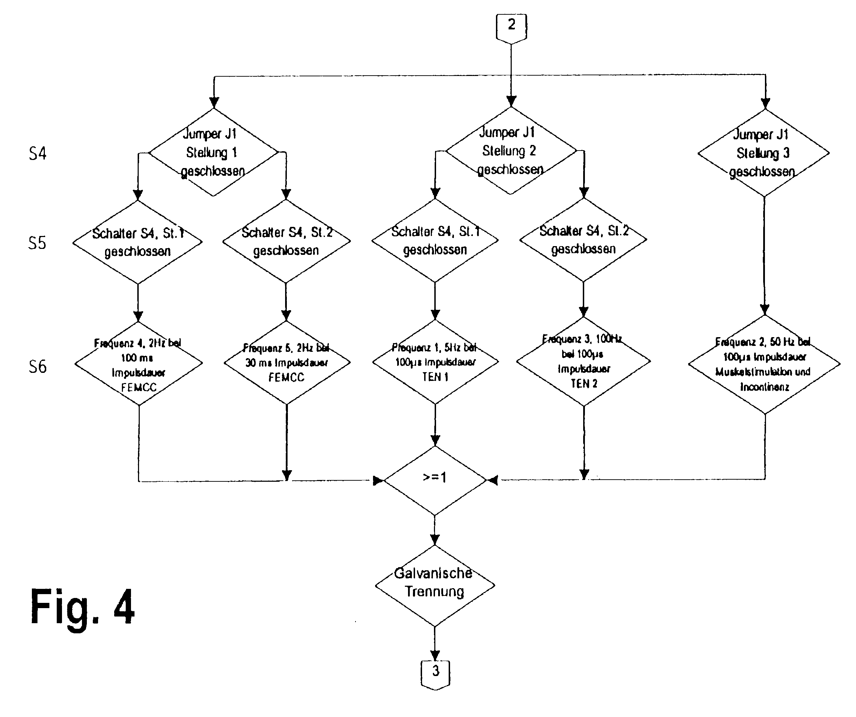

Ferner sind auf der Gehäuseoberseite 5 drei Rastschalter zur Wahl vorbestimmter Betriebszustände vorgesehen. Ein erster Schalter (Schalter 7) dient der Wahl einer Betriebsdauer, ein zweiter Schalter (Schalter 8) der Wahl des Betriebszustands und ein dritter Schalter (Schalter 9) der Wahl des Frequenzmusters. Bei einer bevorzugten Ausführungsform, wie nachfolgend im Zusammenhang mit den Figuren 3 und 4 beschrieben, kann der Benutzer wahlweise mit Schalter 2 eine Behandlungsdauer von 15 Minuten, 30 Minuten oder eine unendliche Behandlungsdauer vorgeben und mit Schalter 3 zwischen einem Dauerbetrieb (D) und einem Intervallbetrieb (I) wählen. Alternativ hierzu können auch nur der zweite und dritte Schalter 8 und 9 vorgesehen sein, wobei dann die Betriebsdauer z.B. mit 30 min. fest vorgegeben werden kann.

Ferner kann das Gerät einen Triggereingang 13 aufweisen, an dem beim Triggerbetrieb ein Spannungssignal anliegt. Vorzugsweise sind Batterie und Geräteeingang galvanisch getrennt. Das Spannungssignal wird in üblicher Weise mit Hilfe einer nicht dargestellten Meß- bzw. Kontaktelektrode lokal von einem Muskel- und/oder Nervengewebe abgegriffen. Das abgegriffene Spannungssignal ist dabei entweder intentionsabhängig gesteuert, wobei eine gewisse Körperfunktion bzw. Muskelkontraktion bewußt bewirkt wird, wie beispielsweise die Aktivierung eines Muskels, kann jedoch auch vom Unterbewußtsein gesteuert sein, wie beispielsweise bei der Kontraktion des Muskulus corpus cavernosum (Mcc) zur Einleitung der Erektion des männlichen Gliedes oder bei der isometrischen Kontraktion der Blasenmuskulatur, insbesondere des Blasenschließmuskels, oder des Mastdarmschließmuskels.
Vorzugsweise wird das abgegriffene Spannungssignal geräteintern verstärkt und dient als Triggersignal für den Oszillator. Insbesondere zur Vermeidung von Rückkopplungseffekten wird geräteintern ein Schwellenwert vorgegeben, so daß das nachfolgend beschriebene Frequenzmuster nur dann über die Kontaktelektroden 1 an das gleiche oder an ein anderes Muskel- und/oder Nervengewebe ausgegeben wird, wenn das verstärkte Spannungssignal, ggf. nach Filterung zur Vermeidung eines Rauschens, diesen Schwellenwert übersteigt.

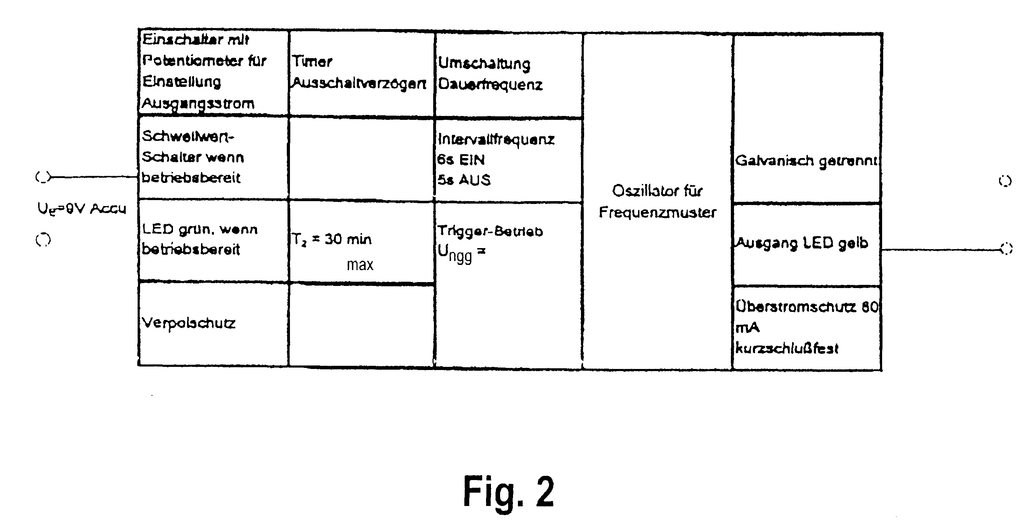

Fig. 2 faßt die wesentlichen, funktionsbeteiligten Elementgruppen und Funktionen eines erfindungsgemäßen Gerätes übersichtlich zusammen.

Zur Stimulation haben sich Frequenzmuster als besonders wirksam herausgestellt, die aus einer Folge von trapezförmigen Stromimpulsen bestehen. Dabei beträgt die Anstiegszeit der Rechteckimpulse bevorzugt etwa 25% der Gesamtimpulsdauer. Bevorzugt wird eine im Vergleich zur Anstiegszeit sehr kleine Abfallzeit. Bei einer weiteren Ausführungsform kann jedoch auch eine in etwa symmetrische Impulsform eingesetzt werden. Bevorzugt wird ein relativ kleines Austastverhältnis (d.h. Verhältnis der Impulsdauer zum Abstand aufeinanderfolgender Impulse) der Stromimpulse unterhalb von 0.25 verwendet. Bevorzugt können die folgenden Betriebszustände in Form von vorbestimmten Frequenzmustern gewählt werden:
1) Impulse mit einer Frequenz von 5 Hz und einer Impulsdauer von 100 Mikrosekunden, vorzugsweise zur transcutanen Elektronervenstimulation, insbesondere zur Stimulation des Musculus temporalis oder bei Schmerzen wie Kopfschmerzen, Nervenschmerzen oder Migräne;
2) Impulse mit einer Frequenz von 50 Hz und einer Impulsdauer von 100 Mikrosekunden, vorzugsweise zur Stimulation von Muskelpartien, insbesondere nach einem Schlaganfall;
3) Impulse mit einer Frequenz von 100 Hz und einer Impulsdauer von 100 Mikrosekunden, vorzugsweise zur transcutanen Elektronervenstimulation, insbesondere zur Stimulation des Musculus temporalis oder bei Schmerzen wie Kopfschmerzen oder Migräne;
4) Impulse mit einer Frequenz von 2 Hz und einer Impulsdauer von 100 Millisekunden, vorzugsweise zur funktionellen Elektromyostimulation des Muskulus corpus cavernosum;
5) Impulse mit einer Frequenz von 2 Hz und einer Impulsdauer von 30 Millisekunden, vorzugsweise zur funktionellen Elektromyostimulation des Muskulus corpus cavernosum.

Dabei haben die Impulse erfindungsgemäß eine Anstiegszeit, welche im Bereich von etwa 10% bis 40% der Gesamtimpulsdauer liegt.

Fig. 3 stellt ein Funktionsschema zur Festlegung eines vorbestimmten Frequenzmusters dar.

In Schritt S1 gibt der Benutzer über die Stellung des Schalters 7 vor, ob er eine Dauerbetrieb wünscht (Stellung 3 des Rastschalters) oder eine zeitlich begrenzte Betriebsdauer von beispielsweise 15 Minuten (Stellung 1) oder 30 Minuten (Stellung 2).

In Schritt S2 wird abgefragt, in welcher Stellung sich der Wechselschalter 5 (nicht dargestellt) am Triggereingang 13 befindet. In Stellung 5.1. liegt ein Dauer- oder Intervallbetrieb vor, während in Stellung 5.2. ein Triggerbetrieb gewählt wird.

In Schritt S3 wird je nach Stellung des Schalters 8 entweder ein Dauerbetrieb gewählt (Stellung 1) oder ein Intervallbetrieb (Stellung 2). Für den Triggerbetrieb ist Schalter 8 ohne Bedeutung.

Bei einer bevorzugten Ausführungsform bedeutet ein Intervallbetrieb, daß das Gerät während 6 Sekunden das Frequenzmuster ausgibt, gefolgt von einem Pauseninterval von 5 Sekunden. Über die Geräteelektronik können jedoch auch andere Zeiten vorgegeben bzw. verstellt werden. Im Dauerbetrieb wird das gewählte Frequenzmuster ständig ausgegeben. Im Triggerbetrieb wird gemäß dem gewählten Betriebszustand entweder ein einziger Stromimpuls oder ein vorbestimmte Anzahl von Stromimpulsen ausgegeben.
Bei einer bevorzugten Ausführungsform sind in die Geräteelektronik einige bevorzugte Frequenzmuster wie beispielsweise die zuvor beschriebenen Frequenzmuster 1) bis 5) fest einprogrammiert. Hierzu dient bevorzugt eine anwenderspezifische Schaltung oder ein ASIC. Der Betriebszustand wird bevorzugt über Verstellung von geräteinternen Jumpern verstellt. Dies geschieht insbesondere aus Sicherheitsgründen, da das Gerät vom Benutzer nur zu einem fest vorgebbaren Benutzungszweck erworben werden soll und eine unbeabsichtigte Verstellung der Betriebszustände wirksam vermieden werden soll. Es kann jedoch auch vorgesehen sein, daß diese Jumper durch Gehäuseschalter ersetzt werden, so daß ein Benutzer den Betriebszustand ohne Öffnen des Gerätes vorgeben oder ändern kann.

Bevorzugt umfaßt die Geräteelektronik einen Jumper J1, der bei den Schritten S4 und S5 den Betriebszustand gemäß Fig. 4 gemeinsam mit den Geräteschaltern weiter festlegt. Befindet sich J1 in einer ersten Stellung, so ist das Gerät für die funktionelle Elektromyostimulation des Muskulus corpus cavernosum (FEMCC) geeignet ausgelegt, wobei die Impulsdauer von der Stellung des Schalters 4 abhängt (Betriebszustände 4 bzw. 5). Befindet sich J1 jedoch in einer zweiten Stellung, so ist das Gerät zur transcutanen Elektronervenstimulation ausgelegt (Betriebszustände 1 bzw. 3). Befindet sich schließlich J1 in einer dritten Stellung, so ist das Gerät geeignet zur Stimulation von Muskelpartien ausgelegt (Betriebszustand 2).

Eine bevorzugte und nichttherapeutische Anwendung des zuvor beschriebenen Gerätes betrifft ein Verfahren zur Verbesserung der körperlichen Erscheinung einer erwachsenen, männlichen Person.

Hierzu werden um die Peniswurzel, in einem Abstand von 2 - 3 cm, vorzugsweise zwei Streifenkontaktelektroden mit einer Größe von etwa 1 cm x 3 cm angebracht bzw. angeklebt. Anschließend wird an die Kontaktelektroden gemäß dem gewählten Betriebszustand eine Folge von Stromimpulsen angelegt, die selektiv den Muskulus corpus cavernosum stimulieren. Bevorzugt wird die Amplitude der Stromimpulse mit Hilfe des Drehpotentiometers 10 so gewählt, daß ein angenehmes Kribbeln auf der Haut verspürt wird.
Der Muskulus corpus cavernosum ist bei der Erektion des männlichen Gliedes maßgeblich beteiligt, da er die Erektion einleitet und unterhält, indem er selektiv für die Füllung des Schwellkörpers sorgt. Die Stimulation kann im Bedarfsfall oder dann vorgenommen werden, wenn dies von einem Trainingsplan vorgesehen ist. Durch die Stärkung des Muskels wird eine natürliche, spontane Erektion möglich, wodurch die körperliche Erscheinung der männlichen Person im Bedarfsfall verbessert wird, weil die Person männlicher wirken kann. Eine besonders stimulierende bzw. aufbauende Wirkung von Stromimpulsen tritt bei den vorstehenden Frequenzmustern 4) und 5) auf.

Bei anderen bevorzugten Verwendungsformen des Geräts werden die Kontaktelektroden im Bereich des Musculus temporalis der Person angebracht (Abbau von Spannungen und Kopfschmerzen bzw. Migräne), wodurch die körperliche Erscheinung dieser Person wegen der entspannenden Wirkung der Stromimpulse verbessert wird. Andere bevorzugte Verwendungsbzw. Trainingszwecke betreffen die funktionelle Stimulation des Blasen- oder Mastdarmschließmuskels, also von isometrisch kontrahierenden Muskeln. Durch regelmäßige Stimulation bzw. Training wird die Wirksamkeit dieser Muskeln erhöht, wodurch sich ebenfalls die körperliche Erscheinung verbessern läßt.

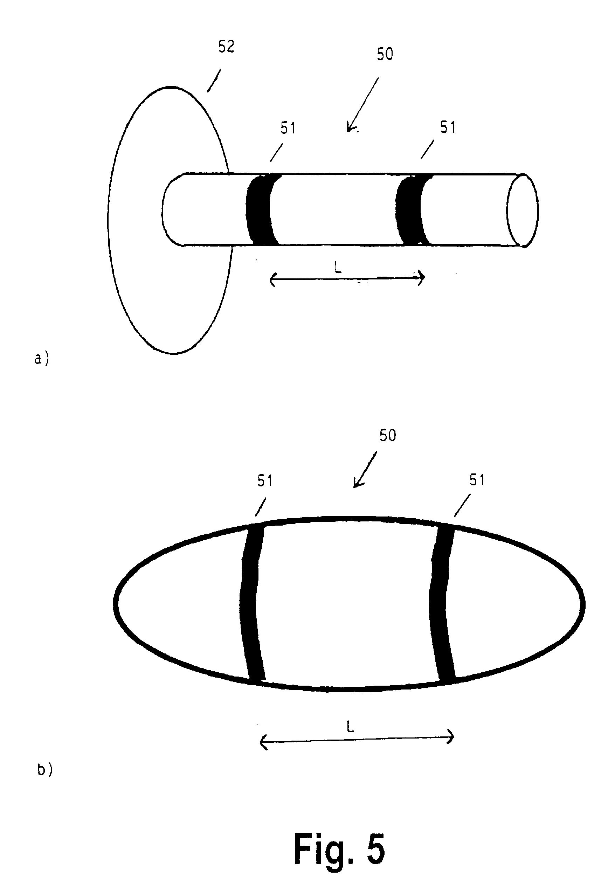

Figur 5 zeigt schließlich zwei bevorzugte Ausführungsformen von Elektrodenkörern, d.h. Körpern, die oberflächlich mit Elektroden versehen sind und zur Einführung in Körperöffnungen bestimmt sind, beispielsweise in den Vaginal- oder Darmbereich. Die Elektroden dienen dabei zum Training bzw. zur Stimulierung von Muskel- oder Nervengeweben in solchen Körperöffnungen, vorzugsweise von Schließmuskelpartien. Bevorzugt werden hierzu zylinderförmige (siehe Figur 5a) oder ellipsenförmige (siehe Figur 5b) Elektrodenkörper. Die Gesamtlänge des Elektrodenkörpers 50 ist zur einfachen Handhabung geeignet ausgelegt, vorzugsweise 10 bis 20 cm.

Zweckmäßig ist der Elektrodenkörper 50 aus einem flexiblen Gummimaterial hergestellt. Vorzugsweise ist der Elektrodenkörper 50 jedoch aufpumpbar und aus einem widerstandsfähigen Material, so daß durch Ändern des Pumpdruckes die Form des Elektrodenkörpers individuell an die Person angepaßt werden kann. Zur Längeneinstellung können Balgabschnitte, vorzugsweise im Mittelbereich, vorgesehen sein.

Die Kontaktelektroden 51 verlaufen in Umfangsrichtung und sind vorzugsweise an einer Stelle unterbrochen, so daß bei einer Änderung des Pumpdruckes bzw. des Umfanges des Elektrodenkörpers 50 die Elektrode selbst nicht zerreißt. Hierzu ist die Elektrode 51 vorzugsweise dehnbar ausgelegt, beispielsweise durch einen Zick-Zackverlauf der Elektrodenbahn in Umfangsrichtung. Der Axialabstand der beiden Elektroden 51 ist an die Ausdehnung des zu trainierenden Muskel- bzw. Nervengewebes angepaßt und beträgt vorzugsweise 1 bis 2 cm. Der Endabstand der beiden Elektroden 51 wird dabei geeignet gewählt. Zur Verstellung des Elektrodenabstands kann der Balgabschnitt (nicht gezeigt) zwischen den beiden Elektroden 51 vorgesehen sein.

Damit der eingeführte Elektrodenkörper 50 nicht in der Körperöffnung verschwindet, wird bevorzugt frühzeitig ein Anschlag 52 auf den Elektrodenkörper aufgesetzt. Dieser Anschlag kann mit dem Elektrodenkörper verschraubt sein, bei einer bevorzugten Ausführungsform ist der Anschlag 52 jedoch als Ring aus Gummi mit einer großen Reibungskonstante ausgelegt, so daß der ringförmige Anschlag 52 seiner Axialverschiebung entgegenwirkt.

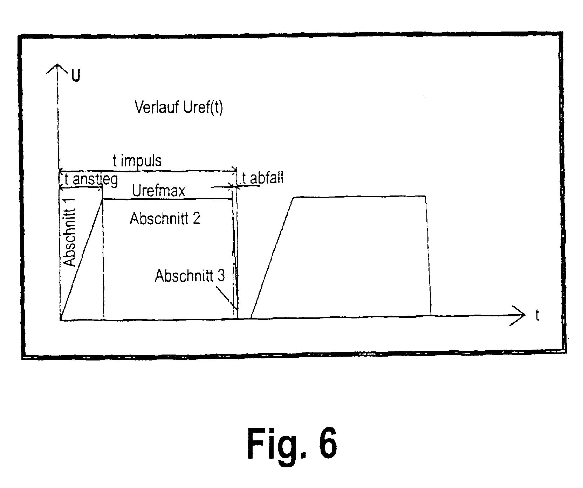

Figur 6 zeigt beispielhaft den Signalverlauf von zwei aufeinanderfolgenden trapezförmigen Impulsen gemäß der vorliegenden Erfindung, wobei eine Abfolge von zwei Impulsen mit positiver Amplitude gezeigt ist. Die Anstiegsflanke des Impulses ist nach der Zeit t anstieg im ersten Abschnitt des Impulses von 0 auf den Maximalwert des Impulses Urefmax angestiegen, worauf diese maximale Signalamplitude Urefmax während der in etwa dreifachen Dauer der Anstiegszeit im zweiten Abschnitt des Impulses gehalten wird. Nach Beendigung des zweiten Abschnittes fällt die Signalamplitude in einer relativ kurzen Zeit, d.h. relativ steil in einem dritten Abschnitt des Signales mit einer im Vergleich zum Gesamtimpuls recht kurzen Zeit t abfall auf 0 ab, wobei nach einer kurzen Austastzeit, welche insgesamt ein kleines Austastverhältnis ergibt, der gleiche Impuls mit entsprechendem Impulsverlauf wieder angelegt wird. Überraschend hat sich gezeigt, daß bei dem Anlegen von Impulsen mit den elektrischen Parametern gemäß der Erfindung eine hohe Erfolgsquote bei der Behandlung erreicht werden kann, d.h. insbesondere eine gute Stimulation von Nervenzellen bzw. Muskelzellen erhalten wurde. Der zweite gezeigte positive Impuls wird mit umgekehrter Polarität an den Patienten angelegt, um einen Gleichstromanteil zu eliminieren.

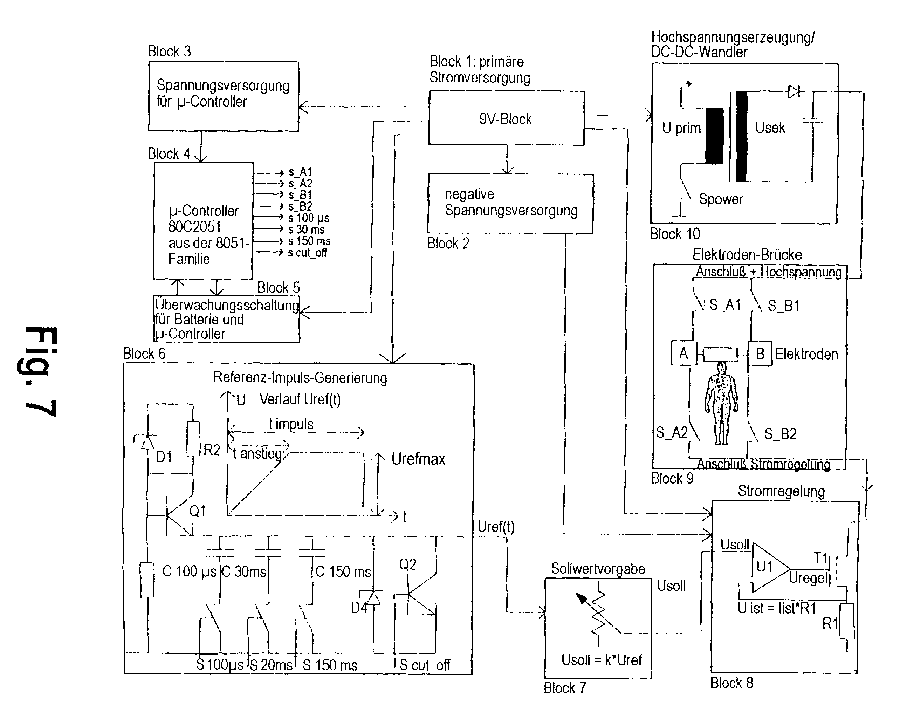

Figur 7 zeigt ein Blockschaltbild der erfindungsgemäßen Vorrichtung, wobei die einzelnen Blöcke nachfolgend beschrieben werden:

Der beispielhaft als primäre Stromversorgung gekennzeichnete 9V-Block stellt die primäre Energieversorgung für die gesamte Schaltung zur Verfügung. Block 2 erzeugt die benötigte negative Spannung, wie z.B. -5V für den Betrieb der Stromregelung, Block 8. Zur Spannungsversorgung für den µ-Controller ist Block 3 vorgesehen, welcher eine Betriebsspannung von z.B. +5V für den µ-Controller, Block 4 erzeugt.

Von dem beispielhaft in Block 4 gezeigten µ-Controller können verschiedene Steueraufgaben übernommen werden, wie z.B. Start und Beendigung der Referenz-Impuls-Generierung bzw. -Erzeugung, Block 6. Hierdurch kann z.B. die Impulsdauer t impuls bestimmt werden. Weiterhin kann der µ-Controller den zur Generierung der Anstiegsflanke notwendigen Kondensator auswählen und für die aktuelle Anstiegsflanke für den Referenz-Impuls aktivieren. Ebenso kann die Elektroden-Brücke, Block 9, angesteuert werden, um eine positive und negative Stromhalbwelle durch Umpolen zu erzeugen.

Block 5 stellt eine Überwachungsschaltung für die Batterie und den µ-Controller dar, von welcher die Funktion des µ-Controllers als Watchdog und die Batteriespannung überwacht wird. So kann z.B. ein Reset ausgelöst werden, wenn der µ-Controller keine Aktivität mehr zeigt. Ebenso kann ein Signal an den µ-Controller ausgegeben werden, wenn die Batteriespannung einen bestimmten Grenzwert unterschreitet, worauf die Elektroden, Block 9, spannungsfrei geschaltet werden, um keine fehlerhaften Impulse abzugeben. Dies kann auch von einer optischen Anzeige signalisiert werden.
Für die Stromregelung, Block 8, kann von der Referenz-Impuls-Generierung, Block 6, ein Referenz-Impuls erzeugt werden. Dieser ist ein Abbild des gewünschten Stromverlaufs. Der Referenz-Impuls kann dabei in Form eines Spannungsverlaufs Uref(t) ausgegeben werden. Dabei kann die Erzeugung des Impulses in drei Abschnitten erfolgen:

### Abschnitt 1: (Anstiegsrampe)

Die Konstantstromquelle D1, R2, Q1 lädt einen Kondensator (C100µs, C30ms, oder C150ms) auf. Der Transistor oder FET befindet sich dabei im gesperrten Zustand: Uref(t) = (Iconst*t)/C
Durch das Zuschalten der einzelnen Kondensatoren werden die Anstiegszeiten für die verschiedenen Impulse (100µs, 30ms, 150ms) festgelegt.

### Abschnitt 2: Begrenzen der Spannung Uref auf Urefmax

Die Zenerdiode D4 begrenzt die Spannung am Kondensator auf Urefmax. Urefmax wird nach Ablauf der Zeit t anstieg erreicht. Die Anstiegszeit t anstieg beträgt 25% von t impuls (100µs, 30ms, 150ms).

### Abschnitt 3: Abfall des Impulses

Der Transistor oder FET Q2 erzeugt die Abfallflanke des Impulses. Der Kondensator wird entladen, indem der FET Q2 durchgeschaltet wird, um eine steile Flanke zu erzeugen. Für eine flachere Flanke können auch entsprechende Kondensatoren verwendet werden.

Ein zweiter Impuls kann unmittelbar darauffolgend in gleicher Weise erzeugt werden. Dieser kann dann z.B. als negativer Stromimpuls an den Benutzer angelegt werden, indem die Elektroden-Brücke, Block 9, umgepolt wird.

Bei Block 7 wird als Eingangssignal das von Block 6 erzeugte Signal Uref(t) angelegt und gelangt auf ein Potentiometer, mit dem der Benutzer die gewünschte Amplitude einstellen kann. Das vom Potentiometer generierte Ausgangssignal, die Ausgangsspannung Usoll, dient als Sollwert für die nachfolgende Stromregelung, Block 8. Für die Ausgangsspannung Usoll gilt somit:
Usoll(t)=k*Uref(t)
Dabei wird der Faktor k durch die Stellung des Potentiometers vorgegeben und kann Werte zwischen 0 und 1 annehmen.

Block 8 stellt die Stromregelung dar und wandelt in Widerstand R1 den durch den Benutzer fließenden Strom in eine proportionale Spannung Uist=R1*(Strom durch den Patienten) um. Der Regler U1 vergleicht diese generierte Spannung mit dem Sollwert Usoll und gibt einen entsprechenden Stellwert Uregel an das Stellglied T1 aus, welches beispielhaft durch einen MosFet gebildet wird. Dieses Stellglied T1 regelt den gewünschten Stromfluß entsprechend.

Das von der Stromregelung, Block 8, festgelegte Stromsignal fließt über die in Block 9 beispielhaft dargestellte Elektroden-Brücke. Dabei kann von dem gezeigten Block 9 die Stromflußrichtung durch den Patienten wie folgt gesteuert werden:
-Stromfluß Elektrode A Õ Elektrode B:
Schalter S_A1 und S_B2 geschlossen, S_A2 und S_B1 offen.
Stromfluß Elektrode B Õ Elektrode A
Schalter S_B1 und S_A2 geschlossen, S_A1 und S_B2 offen.

Dabei können die Schalter auch als Optokoppler ausgeführt und vom µ-Controller, Block 4, angesteuert werden.

Block 10 zeigt einen Hochspannungserzeugungs-DC-DC-Wandler. Von diesem Funktionsblock kann die notwendige Spannung erzeugt werden, um den gewünschten Strom in den Körper des Benutzers einprägen zu können. Dabei kann die Hochspannung mit bekannten Wandlern erzeugt werden, wie z.B. einem Eintakt-Sperrwandler, einem Eintakt-Durchflußwandler oder auch einem Gegentaktwandler. Vorteilhaft erfolgt die Regelung der Hochspannung mit Hilfe eines integrierten Bausteines.

Von der Software des µ-Controllers, Block 4, können weiter folgende Aufgaben übernommen werden:
- Erkennen der Konfiguration des Gerätes, d.h. der aktuellen Betriebsart
- Abfrage des Wählschalters für die Impulsdauer (100µs, 30ms, 150ms) bzw. Frequenz 5 Hz bis 100 Hz
- Abfrage des Wahlschalters für Intervallbetrieb (6sec./10sec.)
- Konfiguration des Funktionsblocks 6, Referenz-Impuls-Generierung anhand der Gerätekonfiguration und der gewählten Impulsdauer bzw. Frequenz
- Starten und Beenden der Referenz-Impuls-Generierung
- Aktivieren und Deaktivieren des Ausgangs
- Abschalten des Ausgangs bei unzureichender Batteriespannung
- Steuern der Elektrodenbrücke, um positive und negative Stromimpulse zu erzeugen
- Überwachen der maximalen Behandlungsdauer

Nachfolgende Tabelle gibt für verschiedene Betriebsarten beispielhaft unter Bezugnahme auf den Signalverlauf von Figur 8A Parameterwerte der Impulse an.

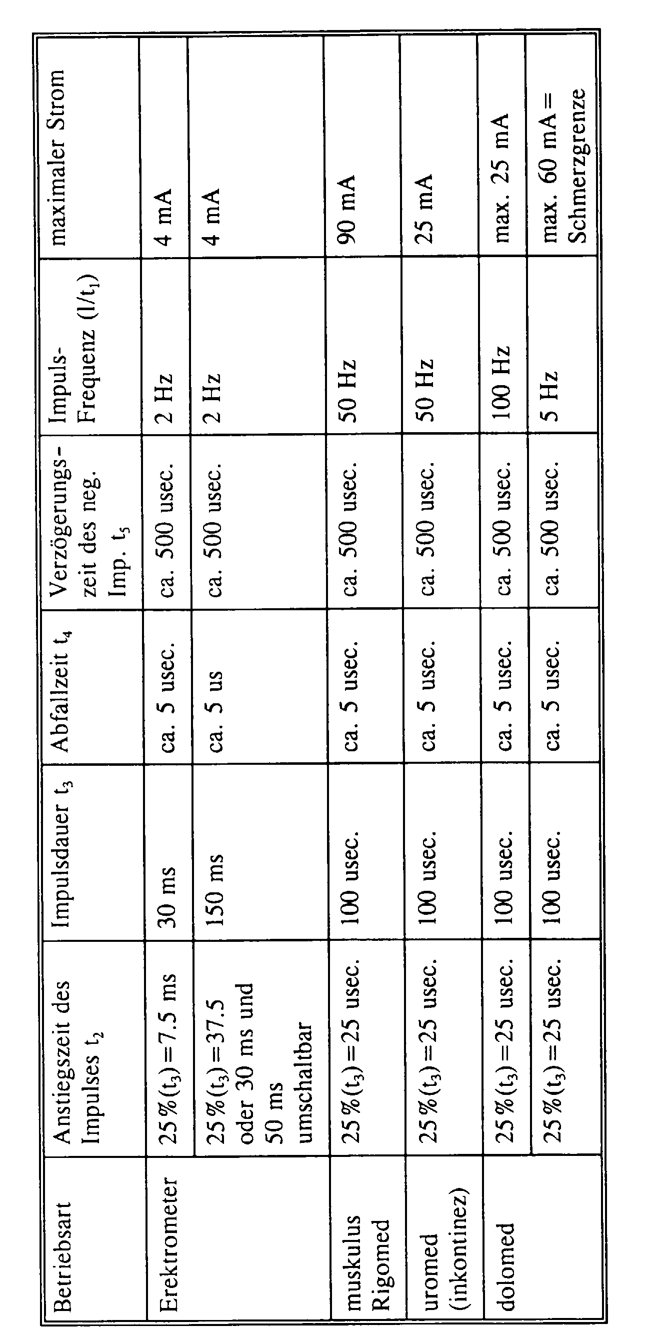

## Patentansprüche

1. Verfahren zum Erzeugen von elektrischen Impulssignalen zur Stimulation von Muskelgewebe und/oder Nervengewebe mit den Schritten:
Erzeugen einer Anstiegsflanke eines Impulses, welche von einem 0-Wert auf den Impulsmaximalwert in einem Zeitraum von 30 µs bis 40 ms ansteigt; und
Halten des Impulsmaximalwertes während einer Haltezeit, die etwa das 1,5-fache bis 9-fache der Dauer der Anstiegsflanke des Impulses beträgt.

2. Verfahren nach Anspruch 1, wobei mehrere Impulse so erzeugt werden, daß diese keinen Gleichstromanteil aufweisen, insbesondere alternierend sind.

3. Verfahren nach Anspruch 1 oder 2, wobei das Austastverhältnis des Impulssignales kleiner oder gleich 0,25 ist, vorzugsweise bei 10⁻⁴ liegt.

4. Verfahren nach einem der vorhergehenden Ansprüche, wobei eine Abfolge der Impulse ununterbrochen erzeugt wird.

5. Verfahren nach einem der Ansprüche 1 bis 3, wobei die Impulse während eines ersten vorgegebenen Zeitraumes erzeugt werden, auf welchen ein zweiter Ruhezeitraum mit vorgegebener Dauer folgt.

6. Verfahren nach einem der vorhergehenden Ansprüche, wobei die Periodendauer der Pulssequenzen im Bereich von etwa 10 ms bis 1 s liegt.

7. Verfahren nach einem der vorhergehenden Ansprüche, wobei die Anstiegszeit der Impulsflanke etwa 25 % der Gesamtdauer des Impulses beträgt.

8. Vorrichtung zur Stimulation von Muskelgewebe und/oder Nervengewebe mit:
mindestens einer Kontaktelektrode, über welche elektrische Impulssignale an Muskelgewebe und/oder Nervengewebe angelegt werden können; und
einer Signalerzeugungsvorrichtung, welche mit der mindestens einen Kontaktelektrode verbunden ist, und elektrische Impulssignale erzeugt, wobei die Impulsdauer der Signale im Bereich zwischen 100 µs und 150 ms liegt, und die Anstiegszeit der Impulssignale etwa 10% bis 40% der Gesamtimpulsdauer beträgt.

9. Vorrichtung nach Anspruch 8, wobei eine Einstellvorrichtung für Parameter der Impulssignale vorgesehen ist.

10. Vorrichtung nach Anspruch 8 oder 9, wobei mindestens eine Meßelektrode zum Abgreifen eines Signals von einer Körperstelle einer Person vorgesehen ist.

11. Vorrichtung nach einem der Ansprüche 8 bis 10, wobei eine Hochspannungserzeugungsvorrichtung für die anzulegenden Impulse vorgesehen ist.

12. Vorrichtung nach einem der Ansprüche 8 bis 11, wobei die Signalerzeugungsvorrichtung Impulse ohne einen Gleichanteil erzeugen kann.

13. Vorrichtung nach einem der Ansprüche 8 bis 12, wobei eine netzunabhängige Energieversorgung vorgesehen ist.

14. Vorrichtung nach einem der Ansprüche 8 bis 13, wobei bei der Vorrichtung eine Betriebsart ein elektrisches Impulssignal nach einem der Ansprüche 1 bis 7 eingestellt werden kann.

15. Verwendung einer elektromedizinischen Vorrichtung nach einem der Ansprüche 8 bis 14 zur gezielten Stimulation bzw. zum Training von Muskel und/oder Nervengewebe.

16. Verwendung einer elektromedizinischen Vorrichtung nach einem der Ansprüche 8 bis 14 zur gezielten Stimulation bzw. Training des Musculus Corpus Cavernosum bei einer erwachsenen, männlichen Person, bzw. zur Verbesserung der körperlichen Erscheinung einer erwachsenen, männlichen Person, wobei die zumindest eine Kontaktelektrode in der Nähe der Peniswurzel angebracht wird, so daß der Musculus Corpus Cavernosum funktionell stimuliert wird, wodurch die körperliche Erscheinung der Person verbessert wird.

17. Verwendung einer elektromedizinischen Vorrichtung nach einem der Ansprüche 8 bis 14 zur Verbesserung der körperlichen Erscheinung einer Person, wobei die zumindest eine Kontaktelektrode am Musculus temporalis der Person angebracht wird, zur funktionellen Stimulation des Musculus temporalis.

18. Vorrichtung nach einem der Ansprüche 8 bis 14, wobei ein Sicherheitsstecker für eine Signalausgangsbuchse vorgesehen ist.
